# EUROPÄISCHE PATENTANMELDUNG

(11) **EP 2 990 531 A1**
(43) Veröffentlichungstag der Anmeldung: **02.03.2016**
(21) Anmeldenummer: 14182526.5
(22) Anmeldetag: 27.08.2014
(51) Int. Cl.: E01C 23/01, E01C 19/48, G01J 5/00

(54) **System für Straßenfertiger mit einer Temperaturmessvorrichtung, Verfahren zur Bestimmung eines Abkühlverhaltens und computerlesbares Speichermedium**

(71) Anmelder: Joseph Vögele AG, 67075 Ludwigshafen (DE)
(72) Erfinder: Buschmann, Martin, 67435 Neustadt (DE); Delius, Henning, 67374 Hanhofen (DE); Rutz, Arnold, 67065 Ludwigshafen (DE)
(74) Vertreter: Grünecker Patent- und Rechtsanwälte PartG mbB

(57) **Zusammenfassung**

System (100) mit einer Temperaturmessvorrichtung (120) zur wiederholten Erfassung von Temperaturwerten eines von einem Fertiger (1) eingebauten Belages (3). Das System (100) ist so konfiguriert, dass ein Temperaturwert für einen bestimmten Messpunkt (351, 351 a, 351 b) zu mindestens zwei unterschiedlichen Zeitpunkten mittels der Temperaturmessvorrichtung (120) ermittelt wird, wobei der bestimmte Messpunkt (351, 351 a, 351 b) in einem Bereich des eingebauten Belages (3) liegt. Das System (100) umfasst ferner eine Auswerteeinheit (140, 140a), die konfiguriert ist, ein Abkühlverhalten des eingebauten Belages (3) zu bestimmen. Dies erfolgt unter Verwendung der mindestens zwei unterschiedlichen Temperaturwerte, die zu den mindestens zwei unterschiedlichen Zeitpunkten für den bestimmten Messpunkt (351, 351 a, 351 b) erfasst wurden.

## Beschreibung

Die Erfindung betrifft ein System mit einer Temperaturmessvorrichtung zur kontinuierlichen oder wiederholten Erfassung von Temperaturwerten eines von einem Straßenfertiger eingebauten Belages und insbesondere ein solches System zum Bestimmen oder Dokumentieren der Einbautemperatur und des Abkühlverhalten des eingebauten Belages.

Ein Straßenfertiger ist eine Maschine, mit der sich ungebundene und gebundene Schichten bzw. Beläge herstellen lassen. Sobald ein eingebauter Belag für die bestimmungsgemäße Benutzung freigegeben wird, sind spätere Nachbesserungen nur mit erheblichem Aufwand, wie beispielsweise der Sperrung von Straßenabschnitten oder Gebäudeteilen, verbunden. Dementsprechend hat die Qualitätskontrolle im Bereich des Asphalteinbaus große Bedeutung. Ein System, das die Messung der Qualität der Asphaltierung oder der Qualität des eingebauten Asphalts erlaubt, zeigt die WO 2004/034351 A2. Insbesondere wird eine manuelle Nachprüfung der Asphalteigenschaften ermöglicht, wobei einzelne Messvorgänge mit Ortsdaten verknüpft werden können.

Eine wesentliche Prozessgröße im Straßenbau ist insbesondere die Verarbeitungstemperatur beim Einbau der verwendeten Beläge, wie etwa Asphalt oder Bitumen. Die Verarbeitungstemperatur hat erheblichen Einfluss auf die Gebrauchseigenschaften wie Standfestigkeit, Schichtenverbund und Lebensdauer der eingebauten Beläge. Straßenfertiger verteilen typischerweise das Asphaltierungsmaterial und führen eine Vorverdichtung einer Decke des Asphaltierungsmaterials mit einer Einbaubohle aus, die am hinteren Ende des Straßenfertigers befestigt ist und von diesem gezogen wird. Der so eingebaute Belag wird nachfolgend von Walzen weiter verdichtet. Die Temperatur des Materials in unterschiedlichen Stufen des Einbauprozesses beeinflusst neben weiteren Faktoren wie beispielsweise den Umgebungs- und Wetterbedingungen beim Einbau den Wirkungsgrad und den Erfolg eines Belagbauauftrags.

Die Verarbeitung beispielsweise von Asphaltierungsmaterial unter optimalen Temperaturbedingungen ist seit Langem als wichtig erkannt worden, ist jedoch oft mit manuellen Kontrollmessungen durch das Unterstützungs- bzw. Bedienpersonal verbunden. Das Asphaltierungsmaterial wird typischerweise mit einer vergleichsweise hohen Temperatur von einer Asphalt- bzw. Bitumenfabrik erhalten. Abhängig von der Entfernung, die eine Liefermaschine zur Baustelle zurücklegt sowie vom Verkehr und der Umgebungstemperatur kann der Asphalt vor der Lieferung abkühlen. Ferner kann der Fortschritt der Asphaltierungsmaschinen und der Verdichtungsmaschinen bzw -walzen variieren. Das Ausmaß der Abkühlung, wenn das Asphaltmaterial schließlich die Asphaltierungsmaschine bzw. den Fertiger erreicht hat, kann abhängig von der Temperatur des Asphaltierungsmaterials bei der Lieferung, von Umgebungsfaktoren, usw. variieren. In manchen Fällen kann sich das Asphaltmaterial in der Asphaltierungsmaschine abscheiden, und somit können vergleichsweise kühlere und vergleichsweise wärmere Taschen bzw. Anhäufungen von Material innerhalb der Maschine existieren, was zu unerwarteten, meist punktuellen, Temperaturgradienten im Asphaltmaterial führt, sobald es auf der Arbeitsoberfläche verteilt ist. Bei einem typischen Einbauprozess wird das Asphaltierungsmaterial ausgestoßen, von der Asphaltierungsmaschine bzw. dem Straßenfertiger verteilt und danach durch die Bohle vorverdichtet, um danach durch die verschiedenen Verdichtungsmaschinen weiter verdichtet zu werden. Hierbei kann die Materialtemperatur signifikant von einer erwarteten Temperatur abweichen. Außerdem kann die Materialtemperatur von einem Abschnitt des eingebauten Belages zum nächsten beispielsweise aufgrund sich ändernder Wetterbedingungen oder aufgrund unbeabsichtigter Abscheidung oder schlechter Vermischung ungleichförmig verteilt sein.

Aufgrund der Wichtigkeit der Einbautemperatur des Belages bei dem Einbauprozess gewinnt das Messen der Einbautemperatur zusehends an Bedeutung und es haben sich in den letzten Jahren unterschiedliche Lösungen herausgebildet, die die Nachfrage nach messtechnischen Nachweisen über die Einbautemperatur befriedigen und dadurch auch Nachbesserungen erleichtern. Bekannte Systeme messen hierfür die Einbautemperatur hinter dem Fertiger, insbesondere hinter der Einbaubohle. Dies reicht vom Pyrometer-Array über Wärmeabtaster bzw. IR-Scanner bis hin zu schwenkbaren Pyrometern. Diese Systeme werden eingesetzt, um einen mehr oder weniger flächigen Eindruck des Temperaturverlaufs hinter dem Fertiger zu gewinnen.

Andere Systeme zur Gewinnung der Einbautemperaturinformation basieren auf Daten einer Infrarotkamera, deren Bilddaten durch eine entsprechende Software zu Abtastzeilen verrechnet werden. Diese Zeilen des ermittelten Wärmebildes zeigen den Temperaturverlauf in einem gewissen Abstand zum Fertiger oder zur Hinterkante der Bohle. Hierbei kann jede Zeile den zur Fahrtrichtung des Straßenfertiges transversalen Temperaturverlauf der eingebauten Schicht repräsentieren, nachdem die einzelnen Zeilen zu einem flächigen Bild, einem sog. Temperaturteppich bzw. zweidimensionalen Temperaturprofil zusammengesetzt worden sind. Diese Bilder können nachfolgend zur Beurteilung der Temperaturverteilung des eingebauten Asphalts verwendet werden. Wie oben erwähnt, ist hierbei eine möglichst gleichmäßige Temperaturverteilung ein Qualitätsmerkmal, da so gleichmäßige Voraussetzungen für eine nachfolgende Walzverdichtung gegeben sind.

Die WO 00/70150 A1 zeigt ein entsprechendes Temperaturüberwachungssystem, das auf einem Straßenfertiger befestigt ist und die Temperatur des eingebauten Belages zeilenweise abtastet. Die gewonnenen Temperaturdaten werden entweder direkt zur Steuerung der Einbaubohle verwendet oder an andere Maschinen des Asphaltierungszuges im Einbauprozess kommuniziert.

Die DE 10 2008 058 481 A1 beschreibt eine Bewertung eines solchen Temperaturverlaufs während des Asphaltierungsprozesses. Insbesondere ist die Anpassung des Asphaltierungsprozesses für die einzelnen Maschinen des Asphaltierungszuges beschrieben. So wird insbesondere auf Basis der gewonnenen Temperaturinformationen des eingebauten Asphalts der Abstand zwischen den Verdichtungswalzen und der Asphaltierungsmaschine bzw. dem Straßenfertiger derart angepasst, dass der Asphalt durch die nachfolgenden Verdichtungswalzen nicht innerhalb eines Temperaturbereiches, der als "weiche Zone" benannt ist, bearbeitet wird. Hierzu wird ein Vergleich zwischen vorhergesagten Temperaturen und den tatsächlich gemessenen Temperaturen vorgeschlagen. Bei einer Differenz zwischen vorhergesagter und tatsächlich gemessener Temperatur werden die Maschinen entsprechend abgebremst oder beschleunigt. Zur Vorhersage der Temperatur wird ein Modell benutzt, welches die äußeren Wetterbedingungen, wie etwa die Umgebungstemperatur, verwendet. Wenn das Modell eine vergleichsweise langsame Abkühlung des Asphaltmaterials aufgrund von hohen Umgebungstemperaturen vorhersagt, können die Maschinen vergleichsweise langsamer oder entsprechend weiter voneinander entfernt fahren.

Die Temperaturmessungen der bekannten Verfahren zur Einbauüberwachung lassen jedoch keinen direkten Rückschluss auf das tatsächliche Abkühlverhalten des eingebauten Belags zu. Bei den bekannten Messsystemen, die eine punkt- oder zeilenförmige Abtastung verwenden, wird versucht, das Abkühlverhalten durch Modelle nachzubilden. Die Modelle berücksichtigen dabei äußere Faktoren, wie beispielweise das Wetter. Bei diesen Lösungen sind entsprechend zusätzliche Sensoren, wie Windmesser oder Regenmesser erforderlich und der Anwender muss manuell einen aktuellen Bewölkungsgrad in das System eingeben. Zur Verbesserung der Qualität der Messergebnisse und zur Vereinfachung des Verfahrens ist es daher wünschenswert, auf solche zusätzlichen Sensoren und manuellen Eingaben verzichten zu können.

Eine direkte Messung oder Bestimmung des Abkühlverhaltens des einbauten Belags ist mit den bekannten System jedoch nicht möglich. So ist selbst bei den Systemen mit kontinuierlicher zeilenweiser Erfassung der Einbautemperatur, um ein flächiges Temperaturbild des Einbaus zu gewinnen, kein Rückschluss auf das Abkühlverhalten oder die Abkühlgeschwindigkeit des Einbaus möglich, da die Zeilen des Wärmebildes bei kontinuierlichem Einbau zum gleichen Zeitpunkt und mit gleichem Abstand zur Bohle aufgenommen wurden.

Deshalb liegt der vorliegenden Erfindung die Aufgabe zugrunde, ein verbesserten System zur Erfassung von Temperaturwerten eines von einem Fertiger eingebauten Belages mittels einer Temperaturmessvorrichtung bereitzustellen, welches insbesondere eine Verbesserung der Messergebnisses ermöglicht, die Anzahl zusätzlich erforderlicher Sensoren verringert oder die Anzahl manuell einzugebender Parameter verringert.

Diese Aufgabe wird durch die Merkmale des Anspruchs 1 gelöst. Ein entsprechender, erfindungsgemäßer Fertiger und ein entsprechendes, erfindungsgemäßes Verfahren sowie ein computerlesbares Speichermedium mit Anweisungen eines entsprechenden, erfindungsgemäßen Steuerverfahrens sind durch die Merkmale der weiteren unabhängigen Ansprüche 13, 14 und 15 gezeigt. Bevorzugte Ausführungsformen der Erfindung sind durch die weiteren Merkmale der Unteransprüche beschrieben.

Die Erfindung betrifft insbesondere ein System mit einer Temperaturmessvorrichtung zur wiederholten Erfassung von Temperaturwerten eines von einem Fertiger eingebauten Belages. Das System ist hierbei konfiguriert, einen Temperaturwert für einen bestimmten Messpunkt im Bereich des eingebauten Belages zu mindestens zwei unterschiedlichen Zeitpunkten mittels der Temperaturmessvorrichtung zu erfassen. Das System umfasst ferner eine Auswerteeinheit, die konfiguriert ist, ein Abkühlverhalten des eingebauten Belages zu bestimmen. Dies erfolgt unter Verwendung der mindestens zwei unterschiedlichen Temperaturwerte, die zu den mindestens zwei unterschiedlichen Zeitpunkten für den bestimmten Messpunkt erfasst wurden.

Der jeweilige zeitliche Abstand zwischen den mindestens zwei unterschiedlichen Zeitpunkten kann dabei vorgegeben sein, was es der Auswerteeinheit erlaubt, das Abkühlverhalten des eingebauten Belages unter der Verwendung des dann bekannten zeitlichen Abstandes zwischen den mindestens zwei unterschiedlichen Zeitpunkten und den mindestens zwei erfassten Temperaturwerten für den bestimmten Messpunkt zu ermitteln oder vorherzusagen.

Die erfassten Temperaturwerte sind bevorzugt Einbautemperaturwerte, die von der Temperaturmessvorrichtung bei oder nach einer Vorverdichtung durch eine Einbaubohle des Fertigers erfasst werden. Die Temperaturmessvorrichtung kann für diesen Fall so konfiguriert werden, dass sie Temperaturwerte mit jeweils vorgegebenem oder einfach ermittelbarem Abstand zum Fertiger oder zur Einbaubohle erfasst. Das erleichtert es, die genaue Position der Messpunkte zu bestimmen bzw. wiederzuerkennen. So kann ein bestimmter Messpunkt auf Basis seines jeweiligen Abstandes zum Fertiger oder zur Einbaubohle zu den mindestens zwei unterschiedlichen Zeitpunkten während des Einbauprozesses ermittelt bzw. wiedererkannt werden. Da sowohl der zeitliche Abstand der Messungen als die Entfernungsabstände der Messungen bekannt oder ermittelbar sind, lässt sich aus den erfassten Daten die Temperatur über der Zeit darstellen und damit auch ein Maß für die Abkühlung bzw. die Abkühlgeschwindigkeit der Belagoberfläche ermitteln.

Gemäß einer weiteren Ausführungsform der Erfindung ist die Temperaturmessvorrichtung derart konfiguriert, dass sie zu jedem Messzeitpunkt eine Vielzahl von Temperaturwerten in einem zweidimensionalen Bereiches auf der Oberfläche des eingebauten Belages erfassen kann. Dieser zweidimensionale Bereich wird auch als der Erfassungsbereich der Temperaturmessvorrichtung bezeichnet. Somit entsprechen die mindestens zwei unterschiedlichen Zeitpunkte, zu denen die Temperaturmessvorrichtung Temperaturmessungen vornimmt, mindestens zwei unterschiedlichen zweidimensionalen Erfassungsbereichen. Diese zweidimensionalen Bereiche werden so gewählt, dass sie sich überlappen. Ferner wird der bestimmte Messpunkt so gewählt, dass er in dem Überlappungsbereich der mindestens zwei Erfassungsbereiche liegt. Damit wird erreicht, dass für den bestimmten Messpunkt auf dem eingebauten Belag eine mehrfache Temperaturmessung zu verschiedenen Zeitpunkten erfolgt, ohne hierfür jedoch zusätzliche Messungen vornehmen zu müssen.

Gemäß einer weiteren Ausführungsform werden die Temperaturwerte mittels der Temperaturmessvorrichtung zeilenweise erfasst, so dass jede erfasste Zeile einen zur Fahrtrichtung des Fertigers transversalen Temperaturverlauf des eingebauten Belages repräsentiert. Bei einem zweidimensionalen Erfassungsbereich der Temperaturmessvorrichtung können zudem zu jedem Messzeitpunkt die Temperaturwerte mehrerer Zeilen gleichzeitig erfasst werden.

Gemäß einer weiteren Ausführungsform der Erfindung umfasst das System oder die Temperaturmessvorrichtung Mittel zur Anbringung an den Fertiger oder an die Einbaubohle. Die Erfassung der Temperaturwerte mittels der Temperaturmessvorrichtung erfolgt in kontinuierlicher Weise, in periodischen Abständen oder in Abhängigkeit einer Geschwindigkeit des Einbauprozesses oder des Fertigers. Diese Merkmale erlauben eine einfache Bestimmung der Position der Messpunkte und der zeitlichen Abstände zwischen den Messungen.

Eine weitere Ausführungsform der Erfindung sieht die Messung der Temperaturwerte an einer Vielzahl von bestimmten Messpunkten zu den jeweils mindestens zwei unterschiedlichen Zeitpunkten vor. Eine gleichzeitige Erfassung der Temperaturwerte für mehrere bekannte Messpunkte hat den Vorteil, dass das Abkühlverhalten des eingebauten Belages auf Basis einer Mittelwertbildung der erfassten Temperaturwerte dieser Vielzahl von Messpunkten ermittelt werden kann. Damit kann der Einfluss von Messfehlern oder punktuellen Material- oder Einbaufehlern auf die Ermittlung des Abkühlverhaltens verringert werden. Mögliche Mittelwertbildungen gemäß einer Ausführungsform umfassen ein arithmetisches Mittel, einen Median, ein quadratisches Mittel und/oder ein gewichtetes arithmetischen Mittel für die erfassten Temperaturwerte der Vielzahl von Messpunkten.

Gemäß einer weiteren Ausführungsform der Erfindung ist die Auswerteeinheit konfiguriert, das Abkühlverhalten des eingebauten Belages auf Basis eines vorgegeben Rechenmodells zu ermitteln. Ein solches Rechenmodell kann mindestens einen zusätzlichen Parameter berücksichtigen, wie etwa die Untergrundtemperatur, die Belagdicke, die Umgebungslufttemperatur, die Luftfeuchtigkeit, die Wolkenabdeckung, die Windgeschwindigkeit und/oder eine weitere Belagmaterialeigenschaft, wie die Asphaltzusammensetzung.

Gemäß einer weiteren Ausführungsform der Erfindung ist die Auswerteeinheit weiterhin konfiguriert, eine Steuergröße des Einbauprozesses des Belages zu bestimmen. Die Steuergröße kann eine Geschwindigkeit einer Maschine des Einbauprozesses, ein Start- bzw. Stoppsignal für eine Maschine des Einbauprozesses und/oder eine Förderbandgeschwindigkeit des Einbauprozesses umfassen. Der Einbauprozess kann somit besser an die tatsächliche Temperatur angepasst werden, da das aus direkter Messung ermittelte Abkühlverhalten des Belages zugrunde gelegt wird. Die Qualität des Einbaus ist somit verbessert, weil die Geschwindigkeit des Einbauprozesses in verbesserter Weise an die tatsächliche Einbautemperatur angepasst werden kann.

Die Temperaturmessvorrichtung gemäß einer Ausführungsform der Erfindung umfasst eine Infrarot-Kamera, einen Infrarot-Scanner, ein schwenkbares Pyrometer, ein Pyrometer-Array und/oder eine Zeilenkamera.

Ferner ist ein Fertiger mit einem System oder der Temperaturmessvorrichtung gemäß einer weiteren Ausführungsform der Erfindung vorgesehen. Hierbei ist die Temperaturmessvorrichtung vorteilhafterweise in ein Thermografiemodul integriert. Darüberhinaus kann die Auswerteeinheit in das Thermografiemodul integriert sein. Alternativ ist eine externe Auswerteeinheit vorgesehen. Ferner ist auch ein System oder ein Thermografiemodul gemäß einer weiteren Ausführungsform der Erfindung vorgesehen, welches unabhängig oder losgelöst vom Fertiger ausgeführt ist. Gemäß einer weiteren Ausführungsform der Erfindung ist ein solches System oder Thermografiemodul als Mobilgerät ausgebildet.

Darüberhinaus betrifft die Erfindung ein entsprechendes Verfahren zum Bestimmen des Abkühlverhaltens sowie ein computerlesbares Speichermedium mit computer-ausführbaren Anweisungen, die bei ihrer Ausführung ein entsprechendes Steuerverfahren ausführen. Erfindungsgemäße Ausführungsformen werden anhand der nachfolgenden Zeichnungen beschrieben. Es zeigen:
- Fig. 1: ein System mit einer Temperaturmessvorrichtung gemäß einer Ausführungsform der Erfindung,
- Fig. 2: einen Straßenfertiger zur Erfassung der Einbautemperatur des Belages während des Einbauprozesses gemäß einer Ausführungsform der Erfindung,
- Fig. 3: einen zweidimensionalen Temperaturverlauf des eingebauten Belages, welcher zeilenweise zusammengesetzt wurde,
- Fig. 4A, 4B und 4C: unterschiedliche, zweidimensionale Bereiche zur Erfassung von Temperaturwerten mittels einer Temperaturmessvorrichtung zu unterschiedlichen Zeitpunkten gemäß einer Ausführungsform der Erfindung,
- Fig. 5: die unterschiedlichen, zweidimensionalen Erfassungsbereiche der Figuren 4A, 4B und 4C,
- Fig. 6: ein Verfahren zum wiederholten Erfassen von Temperaturwerten und zum Bestimmen eines Abkühlverhaltens gemäß einer Ausführungsform der Erfindung, und
- Fig. 7: ein bei der Ausführung von Anweisungen eines computerlesbaren Speichermediums ausgeführtes Steuerverfahren gemäß einer Ausführungsform der Erfindung.

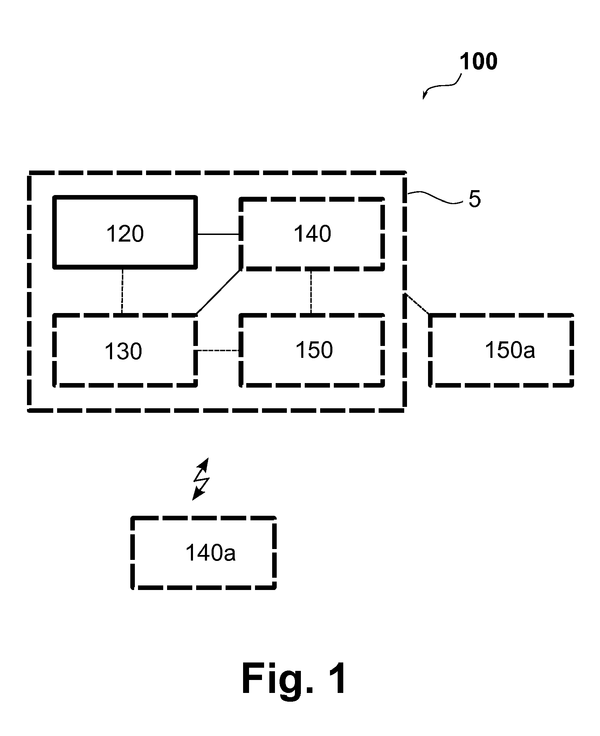

Fig. 1 zeigt ein System 100 gemäß einer Ausführungsform der Erfindung, welches eine Temperaturmessvorrichtung 120 und eine Auswerteeinheit 140 umfasst, wobei die Auswerteeinheit 140 und die Temperaturmessvorrichtung 120 zur Datenübertragung miteinander verbunden sind. Gemäß einer Ausführungsform sind die Temperaturmessvorrichtung 120 und die Auswerteeinheit 140 in einem Thermografiemodul 5 integriert.

Gemäß einer anderen Ausführungsform ist eine Auswerteeinheit 140a außerhalb oder separat vom Thermografiemodul 5 ausgeführt und damit insbesondere entfernt zur Temperaturmessvorrichtung 120 angeordnet. Hierzu kann die Auswerteeinheit 140a mit dem Thermografiemodul 5 mittels einer kabelgebunden oder einer drahtlosen Kommunikationsverbindung Daten austauschen. Im Falle einer externen Auswerteeinheit 140a kann das Thermografiemodul 5 wie auch die Auswerteeinheit 140a ein Schnittstellenmodul (nicht gezeigt) umfassen, welches die Kommunikation und den Datenaustausch mittels eines bekannten Kommunikationsstandards bereitstellt. Bevorzugt werden hierfür bekannte Kommunikationsstandards verwendet, wie etwa Bluetooth, WLAN gemäß einem der Standards IEEE 802.11x (auch als Wi-Fi bezeichnet) oder LAN gemäß einem der Standards IEEE 802.3x (auch als Ethernet bezeichnet). Es können jedoch auch andere Kommunikationsverbindungen wie beispielsweise eine Mobilfunkverbindung verwendet werden. Eine externe Auswerteeinheit 140a hat den Vorteil, dass das Thermografiemodul 5 keine Verarbeitungskapazitäten und Speicherressourcen für die Berechnungen der Auswerteeinheit erfordert. Darüberhinaus kann ein Smartphone, ein PC, ein Laptop, ein Tablet-Gerät oder ein ähnliches Multifunktionsgerät als externe Auswerteeinheit 140a verwendet werden. Hierbei kann eine entsprechende Steuer- und/oder Auswertsoftware, wie beispielsweise ein Anwendungsprogramm oder eine App, auf das externe Gerät 140a aufgespielt bzw. installiert werden.

Alternativ wird die interne Auswerteeinheit 140 oder die externe Auswerteeinheit 140a lediglich als Schnittstelle bzw. Gateway zu einem damit verbunden Server oder zu einem damit verbunden Cloud-Dienst benutzt. Der Server oder der Cloud-Dienst stellt in diesem Fall die Steuer- und/oder Auswertfunktionalität der Auswerteeinheit gemäß der Erfindung bereit. Die hier beschriebene Steuer- und/oder Auswertfunktionalität der Auswerteeinheit gemäß der Erfindung kann auch auf die Auswerteeinheit 140, 140a, einen damit verbunden Server oder Cloud-Dienst und/oder die Temperaturmessvorrichtung 120 verteilt sein.

Die Temperaturmessvorrichtung 120 umfasst bevorzugt eine Infrarotkamera (welche auch als Wärmebild-, Thermografie- oder Thermalkamera bezeichnet wird), die in einem zeilenweisen Abtastmodus operieren kann. Üblicherweise verfügen Infrarotkameras oder Zeilenkameras über die Möglichkeit, ganze Zeilen in einem im Wesentlichen rechteckigen Erfassungsbereich der Kamera zu messen. Eine solche Kamera erlaubt es, einzelne Zeilen auszuwählen, um die entsprechend gemessenen Temperaturwerte dieser einen oder mehreren ausgewählten Zeilen von der Kamera auszugeben. Für die kontinuierliche Temperaturerfassung eines eingebauten Belages ist ein zeilenweises Abtasten des eingebauten Belages vorteilhaft. Zur Dokumentation des Einbauprozesses können die kontinuierlich erfassten Oberflächentemperaturwerte aufgezeichnet werden. Hierzu kann das Thermografiemodul 5 oder die Auswerteeinheit 140, 140a ein Speichermodul (nicht gezeigt) aufweisen. Alternativ oder zusätzlich können die erfassten Daten in geeigneter Form an einen verbunden Server oder Cloud-Dienst zur Speicherung, zur weiteren Bearbeitung oder zur Dokumentation gesendet werden.

Gemäß einer weiteren Ausführungsform der Erfindung ist ein Infrarotscanner bzw. Wärmeabtaster, ein schwenkbares Pyrometer oder ein Pyrometer-Array mit mehreren Pyrometern vorgesehen. Diese können sowohl anstelle der Infrarotkamera 120 als auch zusätzlich zu dieser bereitgestellt werden, wie durch die optionale, zusätzliche Sensorvorrichtung 130 in Fig. 1 dargestellt ist. Im Fall mehrerer Temperaturmessvorrichtungen 120 und 130 beziehen sich die erfassten Temperaturwerte gemäß der beschriebenen Ausführungsformen der Erfindung jeweils auf die erfassten Temperaturwerte dieser Temperaturmessvorrichtungen, auch wenn dies nicht explizit erwähnt ist. Gemäß einer weiteren Ausführungsform der Erfindung ist die optionale Sensorvorrichtung 130 ein zusätzlicher Sensor zur Messung der Untergrund- und/oder Umgebungstemperatur.

Die Auswerteeinheit 140 bzw. 140a ist vorteilhafterweise so konfiguriert, dass ein Abkühlverhalten des eingebauten Belages 3 auf Basis der von der Temperaturmessvorrichtung 120 erfassten Temperaturwerte bestimmt wird. Die Bestimmung erfolgt während des Einbaus und ermöglicht die Einbeziehung des ermittelten Abkühlverhaltens bei der Steuerrung des Einbauprozesses. Aufgrund der Wichtigkeit der Einbautemperatur des Belages während des Einbauprozesses, insbesondere bei der Verdichtung der Schichten durch die Maschinen (Walzen), die dem Fertiger 1 nachfolgen, wird der Einbauprozess mittels der einzelnen Maschinen des Asphaltierungszuges bevorzugt an die Einbautemperatur angepasst. Hierzu gibt es Rechenmodelle oder Nachschlagetabellen, um eine entsprechende Steuergröße des Einbauprozesses aus zuvor ermittelten oder eingegebenen Parametern zu bestimmen. Diese einzugebenden oder zu messenden Parameter sind beispielsweise die Untergrundtemperatur, die Belagdicke, die Umgebungslufttemperatur, die Luftfeuchtigkeit, die Wolkenabdeckung, die Windgeschwindigkeit und/oder weitere Belagmaterialeigenschaften, wie beispielsweise die Asphaltzusammensetzung. Diese Umgebungs- und Wetterfaktoren dienen im Wesentlichen dazu, das Abkühlverhalten abzuschätzen, um sich der tatsächlichen Verarbeitungstemperatur beim Einbau bestmöglich anzunähern und den Einbauprozesses durch entsprechendes Abbremsen oder Beschleunigen der beteiligten Maschinen zu optimieren. Die ermittelten Steuergrößen des Einbauprozesses können die Geschwindigkeit einer Maschine des Einbauprozesses, ein Start- bzw. Stoppsignal für eine Maschine des Einbauprozesses oder eine Förderbandgeschwindigkeit des Einbauprozesses umfassen. Diese Steuergrößen können entsprechend neu festgelegt oder angepasst werden.

Mit der erfindungsgemäßen direkten Ermittlung des Abkühlverhaltens des Belages 3 wird die Anzahl der erforderlichen manuellen Eingaben und zusätzlichen Messung von Umgebungs- und Wetterparametern entweder reduziert oder gänzlich überflüssig. Ebenfalls ermöglicht die Erfindung damit den Verzicht auf die entsprechenden zusätzlichen Sensoren, um die Umgebungs-und Wetterparameter zu messen. Dies vereinfacht die Bedienung während des Einbauprozesses, führt zu weniger komplexen und somit fehlerunanfälligeren Systemen und führt zu einer Verbesserung der Steuerung und Überwachung des Einbauprozesses. Alternativ ist es gemäß einer weiteren Ausführungsform der Erfindung vorgesehen, dass das aus direkter Messung ermittelte Abkühlverhalten zusätzlich zu Umgebungs- und Wetterparametern zur Bestimmung des Temperaturverlaufs und zur nachfolgenden Steuerung des Einbauprozesses verwendet wird. Diese Ausführungsform verbessert weiter die Genauigkeit der Steuerung und Überwachung des Einbauprozesses.

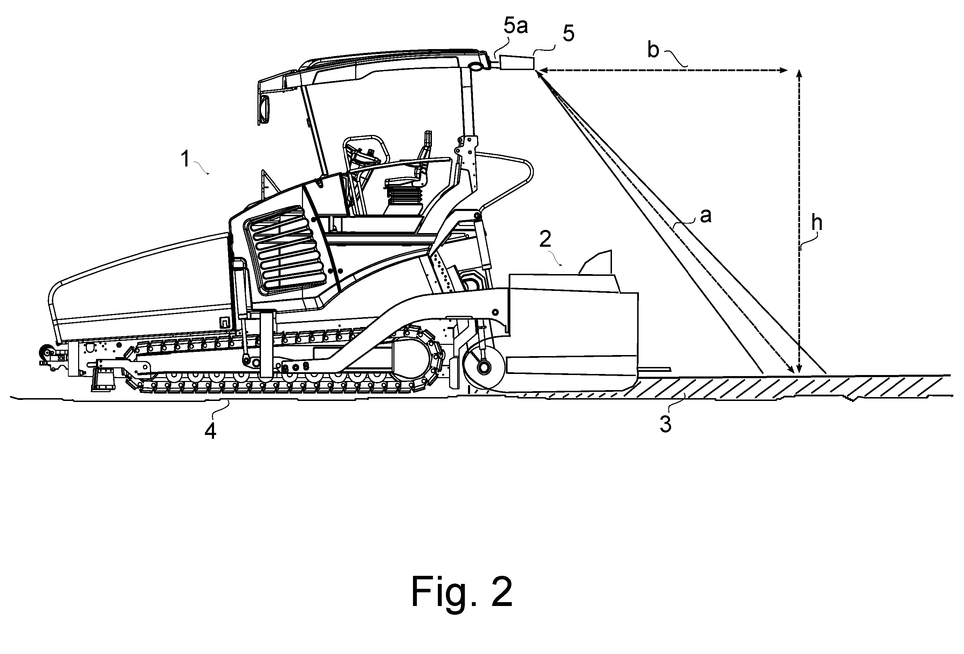

Fig. 2 zeigt beispielhaft einen Straßenfertiger 1 auf einem Planum 4 bei der Aufbringung eines Belages 3, etwa von Asphalt, mittels einer vom Fertiger 1 gezogenen Einbaubohle 2. In der gezeigten Ausführungsform ist am Dach des Fertigers 1 ein Thermografiemodul 5 mittels Fixierungsmitteln 5a angebracht. Dabei befindet sich das Thermografiemodul 5 mit zumindest der Temperaturmessvorrichtung 120 des Systems 100 nach Fig. 1 in einer Höhe h über dem Belag 3 und ist konfiguriert, die Temperatur eines Bereiches des eingebauten Belages 3 in einem Abstand b hinter dem Thermografiemodul 5 zu erfassen, d.h. der Höhe h entsprechend in einem Abstand a vom Thermografiemodul 5. Die Temperaturerfassung kann vorzugsweise zeilenweise in einer zur Fahrtrichtung 300 des Fertigers 1 transversalen Richtung entlang der Breite des eingebauten Belages 3 erfolgen. Es lassen sich somit für jede Temperaturmessung der Temperaturmessvorrichtung 120 die genaue Stelle bzw. der genaue Messpunkt auf dem Belag 3 ermitteln oder festlegen. Alternativ kann das Thermografiemodul 5 oder eine Temperaturmessvorrichtung 120, 130 des Systems 100 an der Bohle angebracht werden.

Da somit sowohl der zeitliche Abstand der Messungen als die Entfernungsabstände der Messungen bekannt oder ermittelbar sind, lässt sich aus den erfassten Temperaturdaten die Temperatur über der Zeit darstellen und damit auch ein Maß für die Abkühlung bzw. die Abkühlgeschwindigkeit der Belagoberfläche ermitteln.

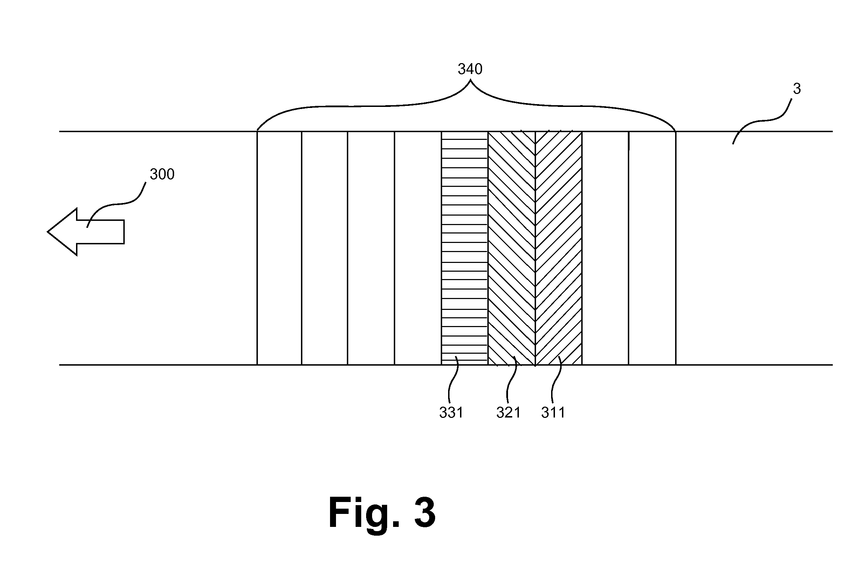

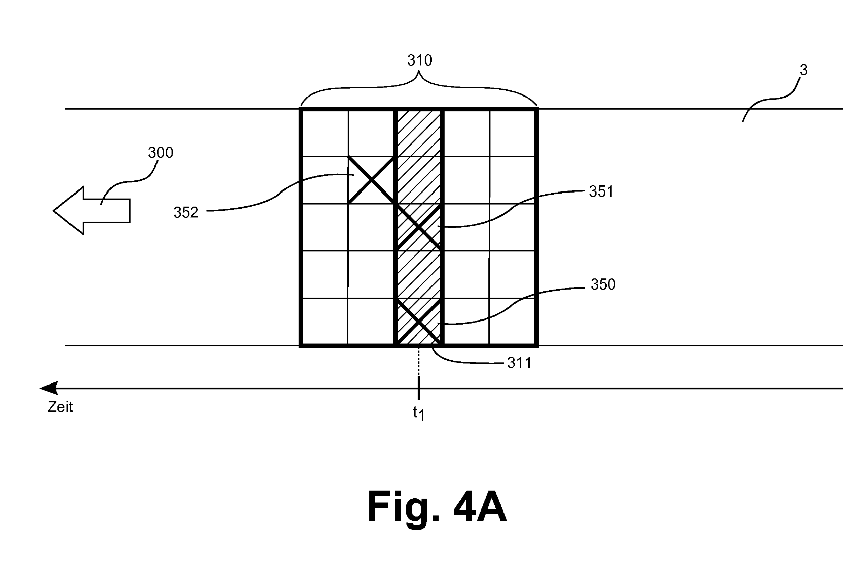

Gemäß einer Ausführungsform wird zudem in vorbestimmten zeitlichen Abständen während der Temperaturerfassung eine Gesamtaufnahme von größeren Bereichen des eingebauten Belages 3 erstellt. Bevorzugt erfolgt dies unter Verwendung einer Temperaturmessvorrichtung 120 zur zeilenmäßigen Abtastung des eingebauten Belages hinter dem Fertiger 1 oder nach der Vorverdichtung durch die Einbaubohle 2. Fig. 3 zeigt beispielhaft eine solche Gesamtaufnahme 340, welche den Temperaturverlauf des eingebauten Belages 3 darstellt, indem einzelne abgetastete Zeilen 311, 321, 331 zu einem flächigen, zweidimensionalen Temperaturprofil oder einem sog. Temperaturteppich zusammengesetzt oder verdichtet wurden. Hierbei sind die mittels der Temperaturmessvorrichtung 120 erfassten Oberflächentemperaturen des Einbaus dargestellt und man kann insbesondere die Temperaturunterschiede zwischen den verschieden Stellen oder Messpunkten des Belages 3 erkennen. In den Figuren 3 und 4A bis 4C ist die Fahrtrichtung 300 des Fertigers 1 von rechts nach links, so dass beispielsweise die Zeile 311 zeitlich vor der Zeile 321 erfasst wurde. Ein solches Gesamtbild des zweidimensionalen Temperaturprofils kann zu Dokumentationszwecken gespeichert oder an einen Server oder einen Cloud-Service gesendet werden.

Die Figuren 4A bis 4C zeigen jeweils die erfassten Temperaturdaten des Belages 3 zu unterschiedlichen Zeitpunkten während des Einbauprozesses oder während der Erfassung durch das Thermografiemodul 5 und dessen Temperaturmessvorrichtung 120. Der in Fig. 4A dargestellte zweidimensionale Bereich 310 wurde von der Temperaturmessvorrichtung 120 wahrend einer Messung zum Zeitpunkt t1 erfasst. Der Bereich 310 zeigt beispielhaft fünf abgetastete Zeilen mit jeweils fünf Messpunkten oder Stellen auf der Oberfläche des Belages 3. Die Anzahl der Zeilen und Messpunkte kann jedoch variieren und hängt insbesondere von der verwendeten IR- bzw. Zeilenkamera ab. Gemäß einer anderen Ausführungsform der Erfindung wird lediglich ein zweidimensionales Temperaturprofil 310 erfasst, ohne explizite Zeilen vorzusehen. Wie in Fig. 4A gezeigt ist, kann die Zeile oder der zweidimensionale Erfassungsbereich 310 der Temperaturmessvorrichtung 120 die gesamte Breite des Belages 3 abdecken. Gemäß einer weiteren Ausführungsform der Erfindung ist dies jedoch nicht gefordert, so dass der Erfassungsbereich 310 der Temperaturmessvorrichtung 120 lediglich einen Teil der Gesamtbreite des Belages 3 abdecken kann, wobei insbesondere die Ränder des Belages 3 ausgespart sein können.

Die Temperaturmessung gemäß dem Beispiel der Fig. 4A erfolgt zu einem Zeitpunkt t1. Gemäß einer Ausführungsform der Erfindung werden zum Zeitpunkt t1 Temperaturwerte einer Zeile 311 erfasst. Dies erfolgt beispielsweise zur Erstellung des zuvor genannten Temperaturteppichs des Belages. Hervorgehoben in Fig. 4A sind beispielhaft drei bestimmte Messpunkte 350, 351 und 352, wobei die Messpunkte 350 und 351 in der Zeile 311 liegen, während der Messpunkt 352 außerhalb der Zeile 311 liegt. In entsprechender Weise können ein oder mehrere Messpunkte 350, 351, 352 in einem erfassten zweidimensionalen Temperaturprofil 310 ohne explizite Zeilenrasterung identifiziert werden. Da die Position und/oder die Ausrichtung des Thermografiemoduls 5 oder der Temperaturmessvorrichtung 120, 130 bekannt sind, kann die genaue Position der Messpunkte 350, 351, 352 auf dem Belag 3 entweder vorgegeben oder ermittelt werden. Im ersten Fall kann die Auswerteeinheit 140 die genaue Position der Messpunkte vorgeben, welche die Temperaturmessvorrichtung 120, 130 nachfolgend zu Messung verwendet. Bei einer externen Auswerteeinheit 140a können die von der Auswerteeinheit vorgegeben Messpunkte an das Thermografiemodul 5 übersendet werden.

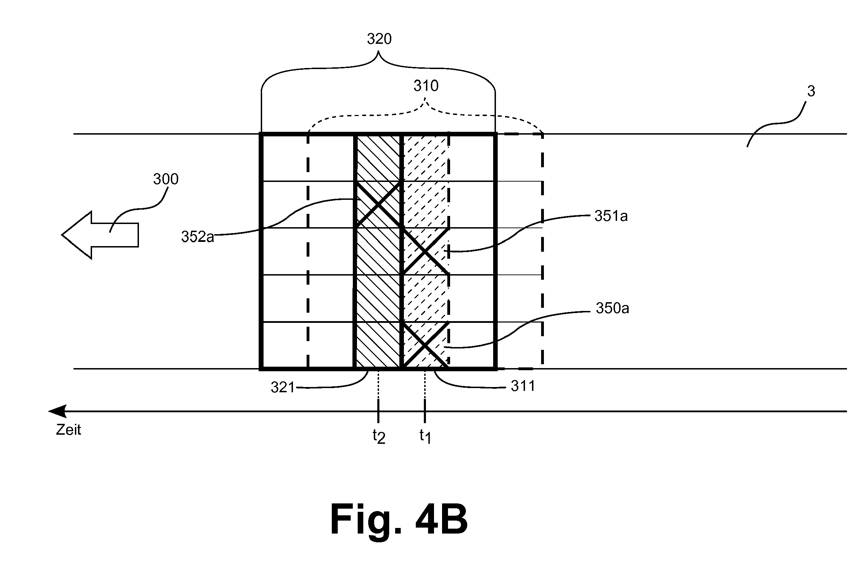

Fig. 4B zeigt entsprechend dem Beispiel der Fig. 4A die Temperaturmessung mittels der Temperaturmessvorrichtung 120 zu einem zweiten Zeitpunkt t2 während des Einbaus, der nach dem Zeitpunkt t1 liegt. Gemäß einer Ausführungsform der Erfindung, welche eine zeilenweise Abtastung vorsieht, betrifft der Zeitpunkt t2 die Erfassung einer zweiten Zeile 321 innerhalb des Erfassungsbereiches 320 der Temperaturmessvorrichtung 120. Gegenüber dem Erfassungsbereich 310 zum Zeitpunkt t1 ist der zweidimensionale Erfassungsbereich 320, der von der Temperaturmessvorrichtung 120 zum Zeitpunkt t2 erfasst wird, entsprechend der Fahrtrichtung 300 des Fertigers 3 weiter nach links gewandert. Hervorgehoben in Fig. 4B sind erneut die drei bestimmten Messpunkte 350, 351 und 352 aus Fig. 4A, welche jedoch nun entgegen der Fahrtrichtung 300 des Fertigers nach rechts wandern. Zur besseren Unterscheidung sind die drei bestimmten Messpunkte in der Fig. 4B mit 350a, 351a und 352a bezeichnet, um zu verdeutlichen, dass diese Messpunkte sich einerseits an den selben absoluten Positionen auf dem Belag 3 wie in Fig. 4A zum Zeitpunkt t1 befinden. Anderseits erfolgt die Temperaturerfassung an diesen bestimmten Messpunkten oder absoluten Positionen zu einer zweiten Zeitpunkt t2 und betreffen einem zweiten Messvorgang. Zur weiteren Verdeutlichung sind in Fig. 4B zusätzlich der erste Messvorgang zum Zeitpunkt t1 und der entsprechende Erfassungsbereich 310 sowie dessen Zeile 311 in gestrichelter Form dargestellt.

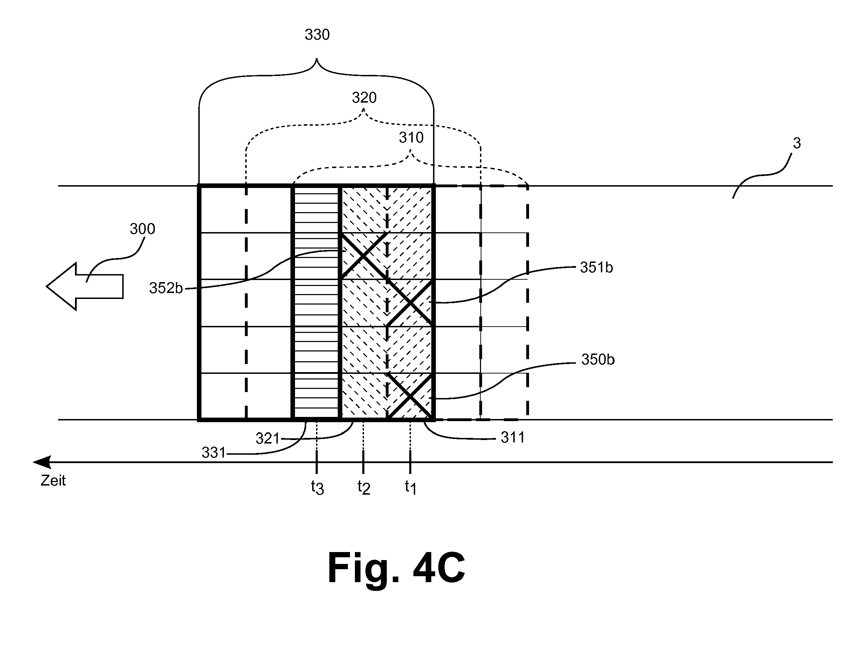

In entsprechender Weise wie Fig. 4B zeigt Fig. 4C einen dritten Messvorgang zum Zeitpunkt t3, der dem Zeitpunkt t2 nachfolgt. Dargestellt ist der zweidimensionale Erfassungsbereich 330 und dessen ermittelte Temperaturverlaufszeile 331 zum Zeitpunkt t3. Im Vergleich mit den Erfassungsbereichen 310 und 320 zu den Zeitpunkten t1 und t2 wandern die drei hervorgehoben Messpunkte 350b, 351 b und 352b zum Zeitpunkt t3 im Erfassungsbereich 330 entgegen der Fahrtrichtung 300 weiter nach rechts, da sich der Fertiger 1 und mit ihm das Thermografiemodul 5 oder die Temperaturmessvorrichtung 120 weiter nach links in Fahrtrichtung 300 bewegen.

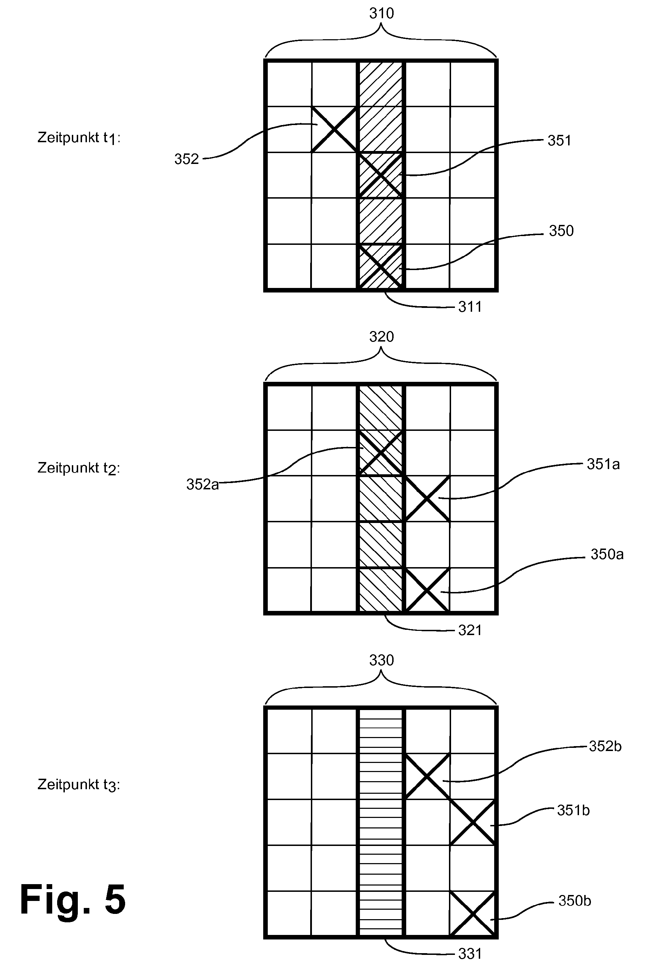

Somit durchlaufen die einzelnen Messpunkte auf dem eingebauten Belag 3 mehrere Aufnahmen der Temperaturmessvorrichtung 120, welche zu unterschiedlichen, aufeinanderfolgenden Zeitpunkten erfasst werden. Die drei Aufnahmen 310, 320, 330 zu den Zeitpunkten t1, t2 und t3 gemäß dem Beispiel der Figuren 4A bis 4C sind in Fig. 5 noch einmal separat gegenübergestellt. Aus diesen unterschiedlichen Temperaturmessungen für ein und denselben Messpunkt auf dem Belag 3, beispielsweise für den Messpunkt 351, 351 a, 351 b, kann ein tatsächliches Abkühlverhalten des eingebauten Belages 3 bestimmt werden. Hierfür werden die zeitlichen Abstände zwischen den unterschiedlichen Temperaturmessungen zu den Zeitpunkten t1, t2 und t3 verwendet. Diese zeitlichen Abstände können zuvor festgelegt sein, beispielweise periodisch, wodurch die Auswerteeinheit 140, 140a die zeitlichen Abstände zwischen den Messungen kennt. Alternativ können gemäß einer anderen Ausführungsform die zeitlichen Abstände der Messungen an den Einbauprozess, insbesondere an die Einbaugeschwindigkeit des zu erfassenden Belages, angepasst werden. In diesem Fall können die zeitlichen Abstände oder die absoluten Messzeitpunkte der Auswerteeinheit 140, 140a mitgeteilt werden.

Gemäß einer Ausführungsform der Erfindung ist ein bestimmter Messpunkt, wie etwa die Stelle 351, bei jeder Messung in Entsprechung zur der tatsächlichen Bewegung der Temperaturmessvorrichtung 120 in Fahrtrichtung 300 vorgegeben oder festgelegt. Hierbei kann die Auswerteeinheit 140, 140a den oder die bestimmten Messpunkte bestimmen oder festlegen und der Temperaturmessvorrichtung 120, 130 oder dem Thermografiemodul 5 mitteilen. Alternativ kann der bestimmte Messpunkt auf dem Belag, wie etwa die Stelle 351, nachträglich aus den gemessenen zweidimensionalen Erfassungsdaten 310, 320 und 330 aufgrund der bekannten Position der Temperaturmessvorrichtung 120, 130 relativ zum Fertiger 1 bzw. Bohle 2 ermittelt werden. Als weitere Alternative ist die Position der Temperaturmessvorrichtung 120, 130 relativ zum Fertiger 1 bzw. Bohle 2 festgelegt und somit eine unveränderbare, bekannte Größe. Die Positionsbestimmung bzw. -festlegung eines bestimmten Messpunktes ist somit zu jeder Zeit möglich, da der Abstand zum Fertiger 1 oder zur Bohle 2, wie zuvor beschrieben wurde, entweder festgelegt ist, von der Auswerteeinheit vorgegeben wurde oder für die Messergebnisse aufgrund bekannter Position und/der Einstellung der Temperaturmessvorrichtung beim Messvorgang ermittelbar ist.

Gemäß einer weiteren Ausführungsform der Erfindung beruht die Ermittlung des Abkühlverhaltens des eingebauten Belages 3 nicht nur auf den Temperarturmessungen an jeweils einem bestimmten Messpunkt 351, 351 a, 351 b. Es können vielmehr bei jeder Temperaturmessung mehrere Messpunkte gleichzeitig betrachtet werden, wie beispielsweise die Messpunkte 350, 351 und 352 zum Zeitpunkt t1. Beispielhaft können alle Messpunkte innerhalb einer Zeile oder verschiedene Messpunkte in unterschiedlichen Zeilen berücksichtigt werden. Die mehreren Messpunkte können hierbei gemäß einer Ausführungsform der Erfindung mittels eines Berechnungsmodells derart kombiniert werden, dass eventuelle Fehlmessungen oder sonstige Ausreißer in den Temperaturmesswerten ausgeglichen werden, herausgefiltert werden oder zumindest ohne erheblichen Einfluss bei der Ermittlung des Abkühlverhaltens und einer entsprechend darauf basierten Steuerung des Einbauprozesses bleiben. Ein einfaches Modell ist die Nichtberücksichtigung bei der Ermittlung des Abkühlverhaltens eines oder mehrerer Messpunkte, die um einen vorgegebenen Betrag über oder unter einem arithmetischen Mittelwert aller Messpunkte liegen. Somit bleiben punktuelle Abweichungen unberücksichtigt, die entweder auf Messfehlern oder Einbaumaterialabweichungen, beispielsweise Einschlüssen, basieren. Alternativ oder zusätzlich kann das Abkühlverhalten gemäß einer weiteren Ausführungsform der Erfindung aufgrund einer Mittelwertbildung der gleichzeitig erfassten Temperaturdaten von mehreren Messpunkten basieren. Die Mittelwertbildung kann beispielsweise ein arithmetisches Mittels, einen Median, ein quadratisches Mittel, und/oder ein gewichteten arithmetischen Mittel für die Temperaturwerte der Vielzahl von gleichzeitig erfassten Messpunkten umfassen.

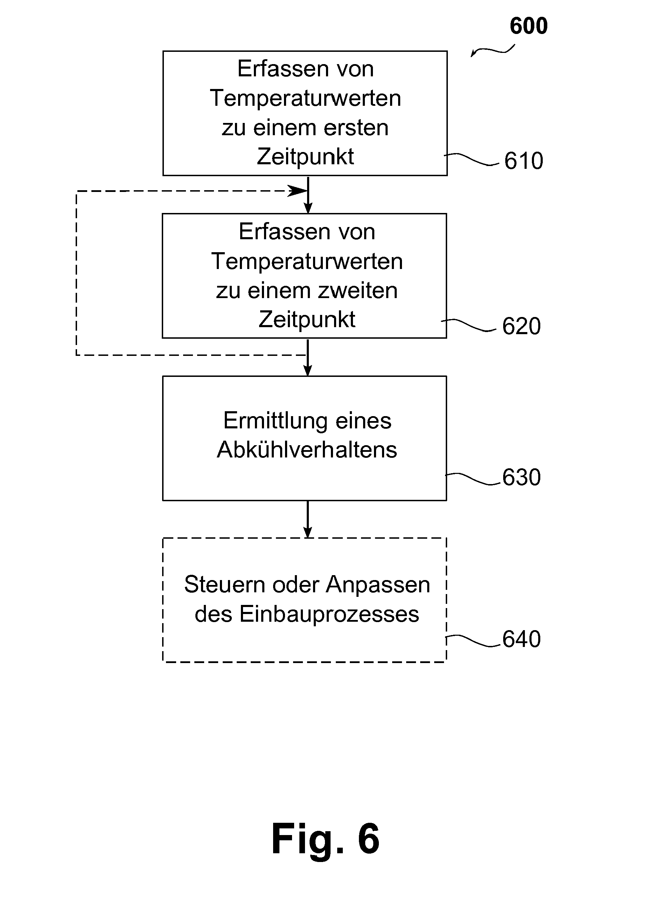

Fig. 6 zeigt schematisch die Schritte eines Verfahrens zum wiederholten Erfassen von Temperaturwerten und zum Ermitteln eines Abkühlverhaltens des von einem Fertiger 1 bzw. Straßenfertiger eingebauten Belages 3 gemäß einer Ausführungsform der Erfindung. Das Verfahren kann von dem zuvor beschriebenen System 100 ausgeführt werden. In Schritt 610 wird zu einem ersten Zeitpunkt (t1) während des Einbauprozesses des Belages die Temperatur eines oder mehrerer bestimmter Messpunkte 351, 351a, 351b von der Temperaturmessvorrichtung 120, 130 erfasst. Wie zuvor beschrieben wurde, liegen der oder die bestimmten Messpunkte im Bereich des eingebauten und von der Temperaturmessvorrichtung 120, 130 erfassten Belages 3 und sind entweder festgelegt, vorgegeben oder ermittelbar. In einem nachfolgenden Schritt 620 werden zu einem zweiten Zeitpunkt von der Temperaturmessvorrichtung 120, 130 erneut Temperaturwerte für den oder die Messpunkte dadurch erfasst, dass der oder die Messpunkte innerhalb des Erfassungsbereiches 320 der Temperaturmessvorrichtung 120, 130 zu dem zweiten Zeitpunkt liegen. Der Schritt 620 kann mehrfach wiederholt werden, so dass weitere Temperaturwerte für den oder die Messpunkte zu weiteren Zeitpunkten erfasst werden können. Die erfassten Temperaturwerte für den oder die bestimmten Messpunkte zu den mindestens zwei unterschiedlichen Zeitpunkten werden anschließend durch die Auswerteinheit 140, 140a in einem Schritt 630 zur Ermittlung des Abkühlverhaltens des eingebauten Belages 3 verwendet. Zusätzlich kann das so ermittelte Abkühlverhaltens gemäß einer weiteren Ausführungsform der Erfindung ausgegeben, gespeichert oder angezeigt werden. Zur Anzeige kann ein Bildschirm an dem Fertiger 1 oder an einem zum Fertiger entfernten Ort vorgesehen sein, wobei der Bildschirm an die Auswerteeinheit 140, 140a oder das Systems 100 angeschlossen ist. Zur Speicherung oder Dokumentierung können die Daten in einem Speicher der Auswerteeinheit 140, 140a oder des Systems 100 gespeichert werden. Ebenfalls können die Daten an einen entfernten Server oder einen Cloud-Dienst übertragen werden.

Gemäß einer Ausführungsform der Erfindung wird das so ermittelte Abkühlverhalten anschließend in einem weiteren Schritt 640 verwendet werden, um beispielsweise den Einbauprozess anzupassen oder zu steuern. Schritt 640 muss nicht von dem System 100 durchgeführt werden und ist optional für das erfindungsgemäße System 100. Das Verfahren gemäß Fig. 6 kann danach von Neuem beginnen oder kontinuierlich während des Einbauprozesses oder der Fortbewegung des Fertiges 1 durchlaufen werden.

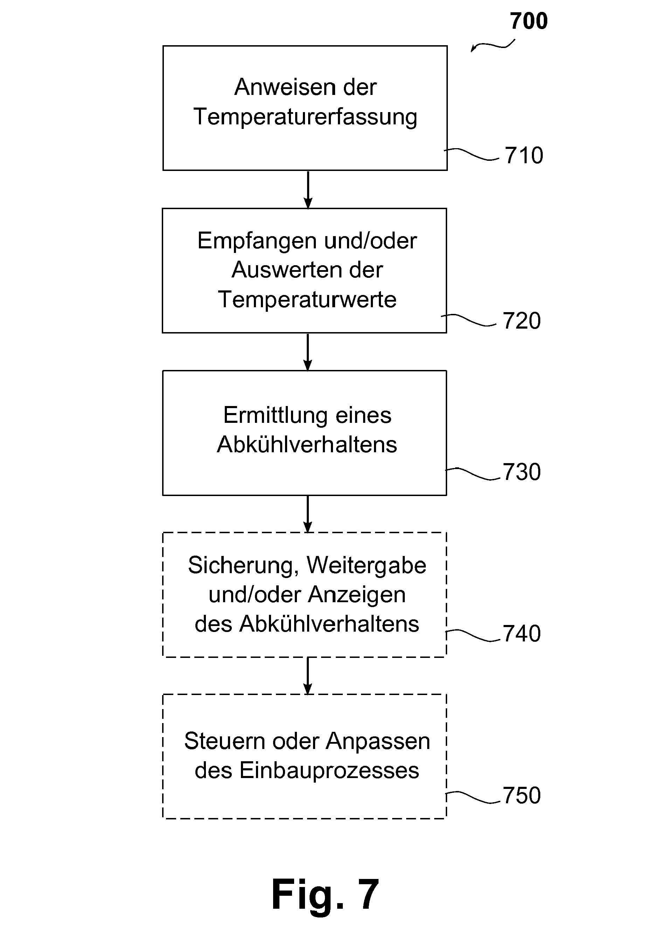

Fig. 7 zeigt Schritte eines entsprechenden Steuerverfahrens 700 gemäß einer weiteren Ausführungsform der Erfindung, das bei der Ausführung von computerausführbaren Anweisungen eines computerlesbaren Speichermediums ausgeführt wird. Zur Ausführung kann die Auswerteeinheit 140 oder 140a dienen oder eine Steuervorrichtung, die mit der Auswerteeinheit 140, 140a mittels einer geeigneten Schnittstelle zur Datenübertragung verbunden ist. Dabei kann die computergestützte Ausführung der computerausführbaren Anweisungen durch eine Steuervorrichtung des Systems 100 innerhalb des Thermografiemoduls 5, an dem Fertiger 1 oder an einem vom Fertiger 1 entfernt angeordneten Ort, ausgeführt werden. Im Schritt 710 des Steuerverfahrens 700 wird eine Temperaturmessvorrichtung 120, 130 oder ein Thermografiemodul 5 angewiesen, während des Einbauprozesses die Temperatur des von einem Fertiger 1 eingebauten Belages 3, wie zuvor beschrieben wurde, zu erfassen. Schritt 710 kann die Anweisung an die Temperaturmessvorrichtung 120, 130 und deren erforderlichen Vorgaben umfassen. In einer weiteren Ausführungsform der Erfindung umfasst Schritt 710 zusätzlich eine oder mehrerer Anweisungen und/oder deren erforderlichen Vorgaben an die Temperaturmessvorrichtung 120, 130 für mehrere Messungen zu den mindestens zwei unterschiedlichen Zeitpunkten (t1, t2, t3). Alternativ können die Messungen zu den mindestens zwei unterschiedlichen Zeitpunkten (t1, t2, t3) als separate Schritte des Steuerverfahrens 700 ausgeführt sein.

Im Schritt 720 werden die Temperaturwerte, welche während des Einbauprozesses von der Temperaturmessvorrichtung 120, 130 erfassten wurden, empfangen oder ausgewertet. Sofern erforderlich, umfasst Schritt 720 gemäß einer Ausführungsform der Erfindung das Empfangen oder Auswerten weiterer Parameter, wie beispielsweise der zeitliche Abstand zwischen den Messungen oder die Position der Messpunkte. Im Schritt 730 des Steuerverfahrens wird das Abkühlverhalten des Belages 3 entsprechend der zuvor beschriebenen Ausführungsformen ermittelt. Im Schritt 740 des Steuerverfahrens wird das ermittelte Abkühlverhalten ausgegeben, gespeichert oder an ein angeschlossenes Empfangsgerät gesendet. Schritt 740 ist hierbei optional und betrifft lediglich die Sicherung, die Weitergabe oder die Anzeige des ermittelten Abkühlverhaltens. Das so ermittelte Abkühlverhaltens kann anschließend in einem weiteren Schritt 750 verwendet werden, um beispielsweise den Einbauprozess anzupassen oder zu steuern. Entsprechende Steuerdaten oder Steuerbefehle können im Schritt 750 oder in einem weiteren Schritt an die entsprechenden Maschinen des Einbauprozesses übertragen werden. Die Schritte 740 und 750 sind hierbei für die Erfindung optional und betreffen lediglich eine weitere Ausführungsform der Erfindung.

Die Verfahren 600 bzw. 700 können gemäß den zuvor beschriebenen Ausführungsformen des Systems 100 oder des Fertigers 1 weiter vorteilhaft ausgebildet sein. In den Figuren 6 und 7 sind die optionalen Schritte gestrichelt dargestellt. Das Verfahren 600 kann entsprechend durch das zuvor beschriebene System 100 verwirklicht und weiter vorteilhaft ausgebildet sein. Das Verfahren 700 kann durch Auswerteeinheit 140, 140a verwirklicht werden und entsprechend weiter vorteilhaft ausgebildet sein. Als weitere Ausführungsform der Erfindung kann das Steuerfahren gemäß Fig. 7 anstatt der zuvor beschriebene Auswerteeinheit 140, 140a verwirklicht werden. In dieser Ausführungsform ist die Auswerteeinheit 140, 140a als Softwareprogramm ausgeführt.

## Patentansprüche

1. System (100) mit einer Temperaturmessvorrichtung (120) zur wiederholten Erfassung von Temperaturwerten eines von einem Straßenfertiger (1) eingebauten Belages (3),
**dadurch gekennzeichnet, dass**
das System (100) konfiguriert ist, einen Temperaturwert für einen bestimmten Messpunkt (351, 351 a, 351 b) zu mindestens zwei unterschiedlichen Zeitpunkten mittels der Temperaturmessvorrichtung (120) zu ermitteln, wobei der bestimmte Messpunkt (351, 351a, 351 b) in einem Bereich des eingebauten Belages (3) liegt, und
das System (100) eine Auswerteeinheit (140, 140a) umfasst, wobei die Auswerteeinheit (140) konfiguriert ist, ein Abkühlverhalten des eingebauten Belages (3) unter Verwendung der mindestens zwei Temperaturwerte, welche für den bestimmten Messpunkt (351, 351a, 351 b) zu den mindestens zwei unterschiedlichen Zeitpunkten erfasst wurden, zu bestimmen.

2. System (100) nach Anspruch 1, wobei der jeweilige zeitliche Abstand zwischen den mindestens zwei unterschiedlichen Zeitpunkten vorgegeben ist, und
wobei die Auswerteeinheit (140, 140a) konfiguriert ist, das Abkühlverhalten des eingebauten Belages (3) unter Verwendung des jeweiligen zeitlichen Abstandes zwischen den mindestens zwei unterschiedlichen Zeitpunkten zu bestimmen.

3. System (100) nach Anspruch 1 oder 2, wobei die Temperaturwerte bei oder nach einer Vorverdichtung durch eine Einbaubohle (2) des Fertigers (1) erfasst werden,
wobei die Temperaturmessvorrichtung (120) konfiguriert ist, Temperaturwerte an Messpunkten im Bereich des eingebauten Belages (3) mit jeweils vorgegebenem oder ermittelbarem Abstand zum Fertiger (1) oder zur Einbaubohle (2) zu erfassen, und
wobei die Position des bestimmten Messpunktes (351, 351 a, 351 b) auf Basis seines jeweiligen Abstandes zum Fertiger (1) oder zur Einbaubohle (2) zu den mindestens zwei unterschiedlichen Zeitpunkten während des Einbauprozesses ermittelt wird.

4. System (100) nach einem der vorigen Ansprüche, wobei die Temperaturmessvorrichtung (120) konfiguriert ist, zu jedem Messzeitpunkt eine Vielzahl von Temperaturwerten in einem zweidimensionalen Bereich (310, 320, 330) auf der Oberfläche des eingebauten Belages (3) zu erfassen, und
wobei die mindestens zwei unterschiedlichen Zeitpunkte zu mindestens zwei unterschiedlichen zweidimensionalen Bereichen (310, 320, 330) korrespondieren, wobei sich die mindestens zwei unterschiedlichen zweidimensionalen Bereiche (310, 320, 330) überlappen und jeder der mindestens zwei unterschiedlichen zweidimensionalen Bereiche (310, 320, 330) den bestimmten Messpunkt (351, 351 a, 351 b) umfasst.

5. System (100) nach einem der vorigen Ansprüche, wobei die Temperaturwerte mittels der Temperaturmessvorrichtung (120) zeilenweise erfasst werden, wobei jede erfasste Zeile (311, 321, 331) einen zur Fahrtrichtung (300) des Fertigers (1) transversalen Temperaturverlauf der eingebauten Belages (3) repräsentiert.

6. System (100) nach Anspruch 4 und 5, wobei die Temperaturmessvorrichtung (120) konfiguriert ist, zu jedem Messzeitpunkt Temperaturwerte mehrerer Zeilen zu erfassen und die mehreren Zeilen eines Messzeitpunkt jeweils einen der zweidimensionalen Bereiche (310, 320, 330) bilden.

7. System (100) nach einem der vorigen Ansprüche, wobei das System (100) oder die Temperaturmessvorrichtung (120) Mittel (5a) zur Anbringung an dem Fertiger (1) oder an der Einbaubohle (2) umfasst und wobei die Temperaturwerte während des Einbauprozesses des Belages (3) mittels der Temperaturmessvorrichtung (120) erfasst werden, und
wobei die Temperaturwerte mittels der Temperaturmessvorrichtung (120) kontinuierlich, in periodischen Abständen oder in Abhängigkeit einer Geschwindigkeit des Einbauprozesses oder des Fertigers (1) erfasst werden.

8. System (100) nach einem der vorigen Ansprüche, wobei das System (100) konfiguriert ist, einen Temperaturwert für jeweils eine Vielzahl von bestimmten Messpunkten (350, 351, 352) zu jedem der mindestens zwei unterschiedlichen Zeitpunkte zu ermitteln, und
wobei die Auswerteeinheit (140, 140a) konfiguriert ist, das Abkühlverhalten des eingebauten Belages (3) auf Basis einer vorgegebenen Mittelwertbildung der Temperaturwerte der Vielzahl von bestimmten Messpunkten (350, 351, 352) zu bestimmen.

9. System (100) nach Anspruch 8, wobei die vorgegebene Mittelwertbildung ein arithmetisches Mittel, einen Median, ein quadratisches Mittel und/oder ein gewichtetes arithmetisehen Mittel für die Temperaturwerte der Vielzahl von bestimmten Messpunkte (350, 351, 352) umfasst.

10. System (100) nach einem der vorigen Ansprüche, wobei die Auswerteeinheit (140, 140a) konfiguriert ist, das Abkühlverhalten des eingebauten Belages (3) auf Basis eines vorgegeben Rechenmodells zu ermitteln, wobei das Rechenmodell mindestens einen zusätzlichen Parameter berücksichtigt und wobei der mindestens eine zusätzliche Parameter eine Untergrundtemperatur, eine Belagdicke, eine Umgebungslufttemperatur, eine Luftfeuchtigkeit, eine Wolkenabdeckung, eine Windgeschwindigkeit und/oder eine Belagmaterialeigenschaft umfasst.

11. System (100) nach einem der vorigen Ansprüche, wobei die Auswerteeinheit (140, 140a) konfiguriert ist, eine Steuergröße des Einbauprozesses des Belages (3) zu bestimmen, wobei die Steuergröße eine Geschwindigkeit einer Maschine des Einbauprozesses, ein Start- oder Stoppsignal für eine Maschine des Einbauprozesses und/oder eine Förderbandgeschwindigkeit des Einbauprozesses umfasst, und
wobei die Auswerteeinheit (140, 140a) des Weiteren konfiguriert ist, die Steuergröße unter Verwendung des ermittelten Abkühlverhaltens des eingebauten Belages (3) zu bestimmen.

12. System (100) nach einem der vorigen Ansprüche, wobei die Temperaturmessvorrichtung (120, 130) eine Infrarot-Kamera, einen Infrarot-Scanner, ein schwenkbares Pyrometer, ein Pyrometer-Array und/oder eine Zeilenkamera umfasst.

13. Straßenfertiger (1) mit einer Temperaturmessvorrichtung (120) oder mit einem System (100) nach einem der vorigen Ansprüche.

14. Verfahren (600) zum Bestimmen eines Abkühlverhaltens eines von einem Straßenfertiger (1) eingebauten Belages (3), wobei das Verfahren umfasst:
Erfassen (610) eines ersten Temperaturwertes für einen bestimmten Messpunkt (351, 351 a, 351 b) zu einem ersten Zeitpunkt während eines Einbauprozesses des Belages (3), wobei der bestimmte Messpunkt (351, 351 a, 351 b) in einem Bereich des von dem Straßenfertiger (1) eingebauten Belages (3) liegt;
Erfassen (620) mindestens eines zweiten Temperaturwertes für den bestimmten Messpunkt (351; 351a; 351 b) zu mindestens einem zweiten Zeitpunkt während des Einbauprozesses, wobei der zweite Zeitpunkt zeitlich nach dem ersten Zeitpunkt liegt;
Bestimmen (630) eines Abkühlverhaltens des eingebauten Belages (3) unter Verwendung der mindestens zwei erfassten Temperaturwerte für den bestimmten Messpunkt (351, 351 a, 351 b).

15. Computerlesbares Speichermedium mit computer-ausführbaren Anweisungen, die bei ihrer Ausführung ein Steuerverfahren (700) durchführen, welches folgende Schritte umfasst:
Anweisen (710) einer Temperaturmessvorrichtung, einen ersten Temperaturwert für einen bestimmten Messpunkt (351, 351 a, 351 b) zu einem ersten Zeitpunkt während eines Einbauprozesses des Belages (3) zu erfassen, wobei der bestimmte Messpunkt (351, 351a, 351 b) in einem Bereich des von dem Straßenfertiger (1) eingebauten Belages (3) liegt;
Anweisen (720) einer Temperaturmessvorrichtung, mindestens einen zweiten Temperaturwert für den bestimmten Messpunkt (351, 351a, 351b) zu mindestens einem zweiten Zeitpunkt während des Einbauprozesses zu erfassen, wobei der zweite Zeitpunkt zeitlich nach dem ersten Zeitpunkt liegt;
Empfangen (730) der mindestens zwei erfassten Temperaturwerte; und
Bestimmen (740) eines Abkühlverhaltens des eingebauten Belages (3) unter Verwendung der mindestens zwei erfassten Temperaturwerte für den bestimmten Messpunkt (351, 351 a, 351 b).

## Geänderte Patentansprüche

### Geänderte Patentansprüche gemäss Regel 137(2) EPÜ.

1. System (100) mit einer Temperaturmessvorrichtung (120) zur wiederholten Erfassung von Temperaturwerten eines von einem Straßenfertiger (1) eingebauten Belages (3),
**dadurch gekennzeichnet, dass**
das System (100) konfiguriert ist, einen Temperaturwert für einen bestimmten Messpunkt (351, 351 a, 351 b) zu mindestens zwei unterschiedlichen Zeitpunkten mittels der Temperaturmessvorrichtung (120) zu ermitteln, wobei der bestimmte Messpunkt (351, 351a, 351 b) in einem Bereich des eingebauten Belages (3) liegt, und
das System (100) eine Auswerteeinheit (140, 140a) umfasst, wobei die Auswerteeinheit (140) konfiguriert ist, ein Abkühlverhalten des eingebauten Belages (3) unter Verwendung der mindestens zwei Temperaturwerte, welche für den bestimmten Messpunkt (351, 351a, 351 b) zu den mindestens zwei unterschiedlichen Zeitpunkten erfasst wurden, zu bestimmen.

2. System (100) nach Anspruch 1, wobei der jeweilige zeitliche Abstand zwischen den mindestens zwei unterschiedlichen Zeitpunkten vorgegeben ist, und
wobei die Auswerteeinheit (140, 140a) konfiguriert ist, das Abkühlverhalten des eingebauten Belages (3) unter Verwendung des jeweiligen zeitlichen Abstandes zwischen den mindestens zwei unterschiedlichen Zeitpunkten zu bestimmen.

3. System (100) nach Anspruch 1 oder 2, wobei die Temperaturwerte bei oder nach einer Vorverdichtung durch eine Einbaubohle (2) des Fertigers (1) erfasst werden,
wobei die Temperaturmessvorrichtung (120) konfiguriert ist, Temperaturwerte an Messpunkten im Bereich des eingebauten Belages (3) mit jeweils vorgegebenem oder ermittelbarem Abstand zum Fertiger (1) oder zur Einbaubohle (2) zu erfassen, und
wobei die Position des bestimmten Messpunktes (351, 351a, 351b) auf Basis seines jeweiligen Abstandes zum Fertiger (1) oder zur Einbaubohle (2) zu den mindestens zwei unterschiedlichen Zeitpunkten während des Einbauprozesses ermittelt wird.

4. System (100) nach einem der vorigen Ansprüche, wobei die Temperaturmessvorrichtung (120) konfiguriert ist, zu jedem Messzeitpunkt eine Vielzahl von Temperaturwerten in einem zweidimensionalen Bereich (310, 320, 330) auf der Oberfläche des eingebauten Belages (3) zu erfassen, und
wobei die mindestens zwei unterschiedlichen Zeitpunkte zu mindestens zwei unterschiedlichen zweidimensionalen Bereichen (310, 320, 330) korrespondieren, wobei sich die mindestens zwei unterschiedlichen zweidimensionalen Bereiche (310, 320, 330) überlappen und jeder der mindestens zwei unterschiedlichen zweidimensionalen Bereiche (310, 320, 330) den bestimmten Messpunkt (351, 351 a, 351 b) umfasst.

5. System (100) nach einem der vorigen Ansprüche, wobei die Temperaturwerte mittels der Temperaturmessvorrichtung (120) zeilenweise erfasst werden, wobei jede erfasste Zeile (311, 321, 331) einen zur Fahrtrichtung (300) des Fertigers (1) transversalen Temperaturverlauf der eingebauten Belages (3) repräsentiert.

6. System (100) nach Anspruch 4 und 5, wobei die Temperaturmessvorrichtung (120) konfiguriert ist, zu jedem Messzeitpunkt Temperaturwerte mehrerer Zeilen zu erfassen und die mehreren Zeilen eines Messzeitpunkt jeweils einen der zweidimensionalen Bereiche (310, 320, 330) bilden.

7. System (100) nach einem der vorigen Ansprüche, wobei das System (100) oder die Temperaturmessvorrichtung (120) Mittel (5a) zur Anbringung an dem Fertiger (1) oder an der Einbaubohle (2) umfasst und wobei die Temperaturwerte während des Einbauprozesses des Belages (3) mittels der Temperaturmessvorrichtung (120) erfasst werden, und
wobei die Temperaturwerte mittels der Temperaturmessvorrichtung (120) kontinuierlich, in periodischen Abständen oder in Abhängigkeit einer Geschwindigkeit des Einbauprozesses oder des Fertigers (1) erfasst werden.

8. System (100) nach einem der vorigen Ansprüche, wobei das System (100) konfiguriert ist, einen Temperaturwert für jeweils eine Vielzahl von bestimmten Messpunkten (350, 351, 352) zu jedem der mindestens zwei unterschiedlichen Zeitpunkte zu ermitteln, und
wobei die Auswerteeinheit (140, 140a) konfiguriert ist, das Abkühlverhalten des eingebauten Belages (3) auf Basis einer vorgegebenen Mittelwertbildung der Temperaturwerte der Vielzahl von bestimmten Messpunkten (350, 351, 352) zu bestimmen.

9. System (100) nach Anspruch 8, wobei die vorgegebene Mittelwertbildung ein arithmetisches Mittel, einen Median, ein quadratisches Mittel und/oder ein gewichtetes arithmetischen Mittel für die Temperaturwerte der Vielzahl von bestimmten Messpunkte (350, 351, 352) umfasst.

10. System (100) nach einem der vorigen Ansprüche, wobei die Auswerteeinheit (140, 140a) konfiguriert ist, das Abkühlverhalten des eingebauten Belages (3) auf Basis eines vorgegeben Rechenmodells zu ermitteln, wobei das Rechenmodell mindestens einen zusätzlichen Parameter berücksichtigt und wobei der mindestens eine zusätzliche Parameter eine Untergrundtemperatur, eine Belagdicke, eine Umgebungslufttemperatur, eine Luftfeuchtigkeit, eine Wolkenabdeckung, eine Windgeschwindigkeit und/oder eine Belagmaterialeigenschaft umfasst.

11. System (100) nach einem der vorigen Ansprüche, wobei die Auswerteeinheit (140, 140a) konfiguriert ist, eine Steuergröße des Einbauprozesses des Belages (3) zu bestimmen, wobei die Steuergröße eine Geschwindigkeit einer Maschine des Einbauprozesses, ein Start- oder Stoppsignal für eine Maschine des Einbauprozesses und/oder eine Förderbandgeschwindigkeit des Einbauprozesses umfasst, und
wobei die Auswerteeinheit (140, 140a) des Weiteren konfiguriert ist, die Steuergröße unter Verwendung des ermittelten Abkühlverhaltens des eingebauten Belages (3) zu bestimmen.

12. System (100) nach einem der vorigen Ansprüche, wobei die Temperaturmessvorrichtung (120, 130) eine Infrarot-Kamera, einen Infrarot-Scanner, ein schwenkbares Pyrometer, ein Pyrometer-Array und/oder eine Zeilenkamera umfasst.

13. Straßenfertiger (1) mit einem System (100) nach einem der vorigen Ansprüche.

14. Verfahren (600) zum Bestimmen eines Abkühlverhaltens eines von einem Straßenfertiger (1) eingebauten Belages (3), wobei das Verfahren umfasst:
Erfassen (610) eines ersten Temperaturwertes für einen bestimmten Messpunkt (351, 351 a, 351 b) zu einem ersten Zeitpunkt während eines Einbauprozesses des Belages (3), wobei der bestimmte Messpunkt (351, 351 a, 351 b) in einem Bereich des von dem Straßenfertiger (1) eingebauten Belages (3) liegt;
**dadurch gekennzeichnet, dass** das Verfahren des Weiteren umfasst:
Erfassen (620) mindestens eines zweiten Temperaturwertes für den bestimmten Messpunkt (351; 351 a; 351 b) zu mindestens einem zweiten Zeitpunkt während des Einbauprozesses, wobei der zweite Zeitpunkt zeitlich nach dem ersten Zeitpunkt liegt;
Bestimmen (630) eines Abkühlverhaltens des eingebauten Belages (3) unter Verwendung der mindestens zwei erfassten Temperaturwerte für den bestimmten Messpunkt (351, 351 a, 351 b).

15. Computerlesbares Speichermedium mit computer-ausführbaren Anweisungen, die bei ihrer Ausführung ein Steuerverfahren (700) durchführen, welches folgenden Schritt umfasst:
Anweisen (710) einer Temperaturmessvorrichtung, einen ersten Temperaturwert für einen bestimmten Messpunkt (351, 351a, 351 b) zu einem ersten Zeitpunkt während eines Einbauprozesses des Belages (3) zu erfassen, wobei der bestimmte Messpunkt (351, 351 a, 351 b) in einem Bereich des von dem Straßenfertiger (1) eingebauten Belages (3) liegt;
**dadurch gekennzeichnet, dass** das Steuerverfahren des Weiteren folgende Schritte umfasst:
Anweisen (720) einer Temperaturmessvorrichtung, mindestens einen zweiten Temperaturwert für den bestimmten Messpunkt (351, 351a, 351 b) zu mindestens einem zweiten Zeitpunkt während des Einbauprozesses zu erfassen, wobei der zweite Zeitpunkt zeitlich nach dem ersten Zeitpunkt liegt;
Empfangen (730) der mindestens zwei erfassten Temperaturwerte; und
Bestimmen (740) eines Abkühlverhaltens des eingebauten Belages (3) unter Verwendung der mindestens zwei erfassten Temperaturwerte für den bestimmten Messpunkt (351, 351a, 351 b).
